# EUROPEAN PATENT SPECIFICATION

(11) **EP 2 251 046 B1**
(45) Date of publication and mention of the grant of the patent: **22.11.2017**
(21) Application number: 10159872.0
(22) Date of filing: 14.04.2010
(51) Int. Cl.: A61L 27/04

(54) **Nanotextured cobalt-chromium alloy articles**
Nanotexturierte Kobalt-Chrom-Legierungsartikel
Articles d'alliage de cobalt chrome nanotexturés

(30) Priority: 15.04.2009 US 169365 P
(43) Date of publication of application: 17.11.2010
(73) Proprietor: DePuy Products, Inc., Warsaw, IN 46581 (US)
(72) Inventor: Tong, Weidong, Warsaw, IN 46580 (US); Salvati, Lawrence, Goshen, IN 46526 (US); Vass, Stephanie A, Warsaw, IN 46582 (US)
(74) Representative: Curran, Clair

(56) References cited:
- EP-A1- 1 736 181

## Description

The invention concerns nanotextured articles made from cobalt chromium alloy and having high wettability and methods for making and using same.

There are a number of design criteria which have long been sought for segmental bone replacement implants including (1) the implant should last the lifetime of the patient without losing function or initiating any adverse process response; (2) the implant should restore the normal function of the bone in which it is implanted; and (3) the implant should be producible on a commercial scale. To satisfy the foregoing criteria, not only should the implant support the imposed load, often of a fluctuating nature, but the interface between the implant and the bone should also withstand the load requirement.

A plastic cement such as polymethyl methacrylate is often used to affix an implant to bone as well as to improve the fit between the implant and the bone. Implants also have been provided with porous coatings which mate with the bone and invite bone ingrowth such that, after a period of time, the prosthesis becomes integrated into the bone structure. Typical such coatings are disclosed in US-3855638, US-4206516, US-4156943 and US-4612160.

Ceramic coatings have also been used to good effect and often are particularly desirable because of the affinity between bone and ceramic materials such as alumina (Al₂O₃). Typical such coatings are disclosed in US-4145764 and US-4483678 which are particularly concerned with dental implants, and US-4309488 and US-4846837 which more broadly disclose implantable bone replacement material for use throughout the body.

Other work has utilized highly convoluted surfaces on the implant. For example, US-5368881 and US-5658333 disclose the use of non-spherical powder to produce a roughened surface for prosthesis. These surfaces, however, are known to have little to no inter-connected porosity. EP 1 736 181 A1 discloses a method for etching the surface of a CoCr implant, the etching solution does not comprise toxic chemicals and comprises both HCl and additional Cl species. The etching solution further comprises an oxidant. In one aspect, the invention provides articles suitable for implantation in a mammal which is formed from an alloy of cobalt and chromium and has an oxide layer on the surface of the alloy that has a thickness of 20 to 40 Å. The surface oxide layer is enriched in chromium relative to the composition of the alloy, and includes a plurality of indentations that, independently, have a diameter of from about 40 to about 500 nm. The oxide layer can have a liquid absorbing capacity of at least about 0.078 µl.mm⁻² (50 µl.in⁻²). In some articles, the cobalt-chromium alloy is CoCrMo. In some embodiments, the surface of said article on which the oxide is formed is provided by beads, especially of cobalt-chromium alloy.

Other aspects of the invention concern methods for making nanotextured articles having high wettability. Some articles have properties as described above. Some methods comprise contacting an article that includes alloy of cobalt and chromium with a solution comprising hydrochloric acid for a time and at a temperature effective to form a surface oxide layer on the alloy that has a thickness of 20 to 40 Å, is enriched in chromium relative to composition of the alloy, and includes a plurality of indentations that, independently, have a diameter of from about 40 to about 500 nm. In certain preferred embodiments, the solution is substantially free of oxidizing agent.

In some processes, the solution has a concentration of H⁺ of at least about 4M and a concentration of Cl⁻ of at least about 4M. The solution can further comprise a chloride-containing compound at a concentration in the range of about 0.01M to 8M. In some embodiments, the solution has a concentration of H⁺ of about 0.8 to about 12M and a concentration of Cl⁻ that is greater than said concentration of H⁺. Preferred chloride-containing compounds include sodium chloride (NaCl), potassium chloride (KCl), calcium chloride (CaCl₂), ammonium chloride (NH₄Cl), ferric chloride (FeCl₃), ferrous chloride (FeCl₂), cobalt chloride (CoCl₂), magnesium chloride (MgCl₂) and mixtures thereof.

Preferably, the article is contacted with the solution at a temperature of at least about 20°C. The temperature of the solution can be up to about 100°C, preferably up to about 40°C. The article can be contacted with the solution for at least about 30 minutes. The article can be contacted with the solution for up to about 96 hours.

Typically, articles provided by the invention are suitable for implantation into a mammal. Some articles are joint replacement prostheses or components thereof. Certain articles are components of a hip or knee joint prostheses.

Embodiments of the invention are described below by way of example with reference to the accompanying drawings, in which:

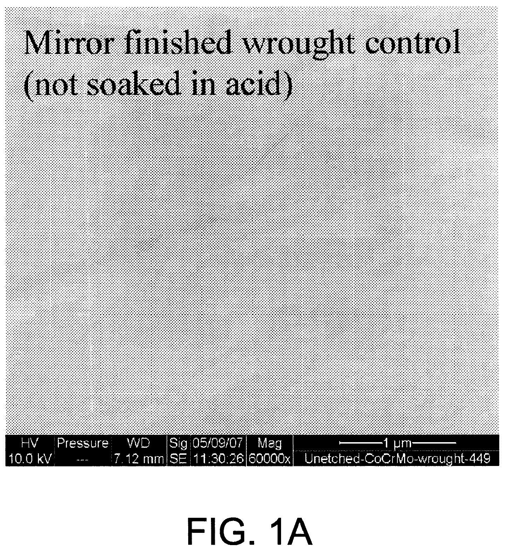

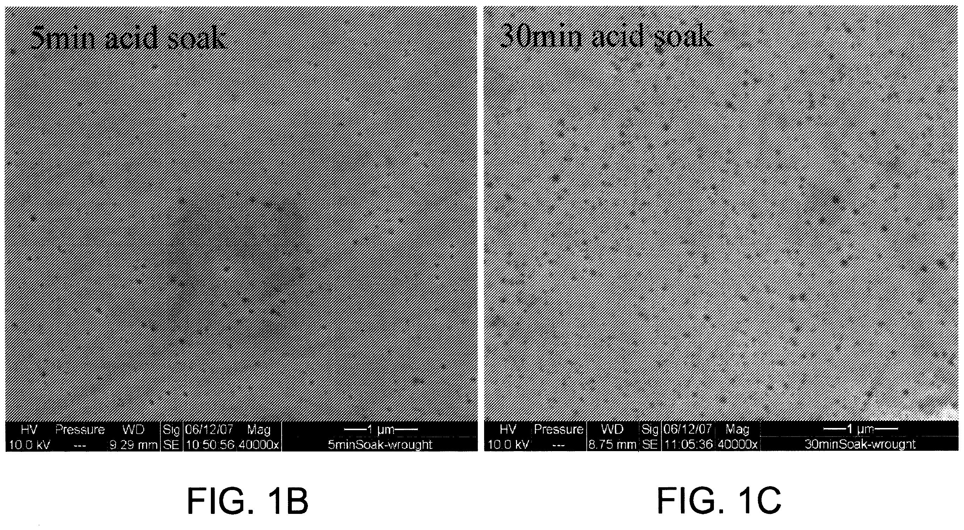

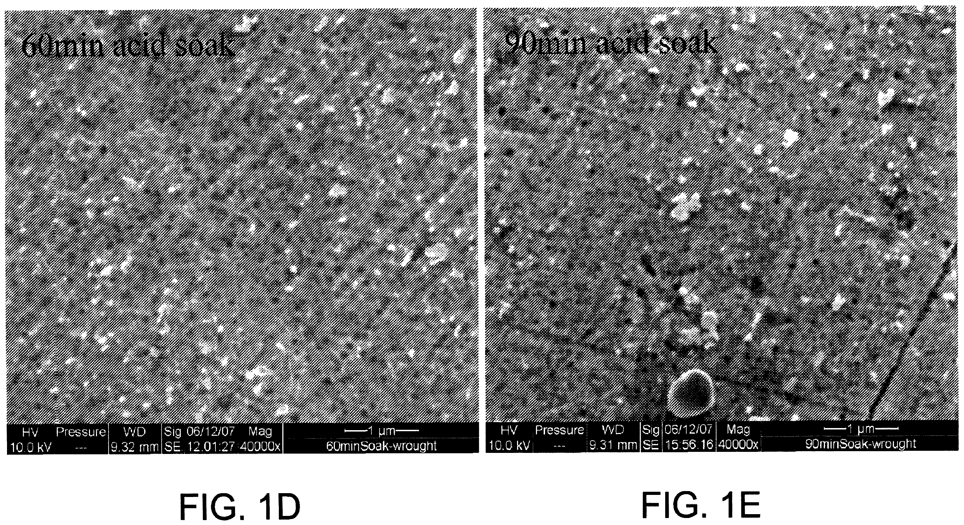

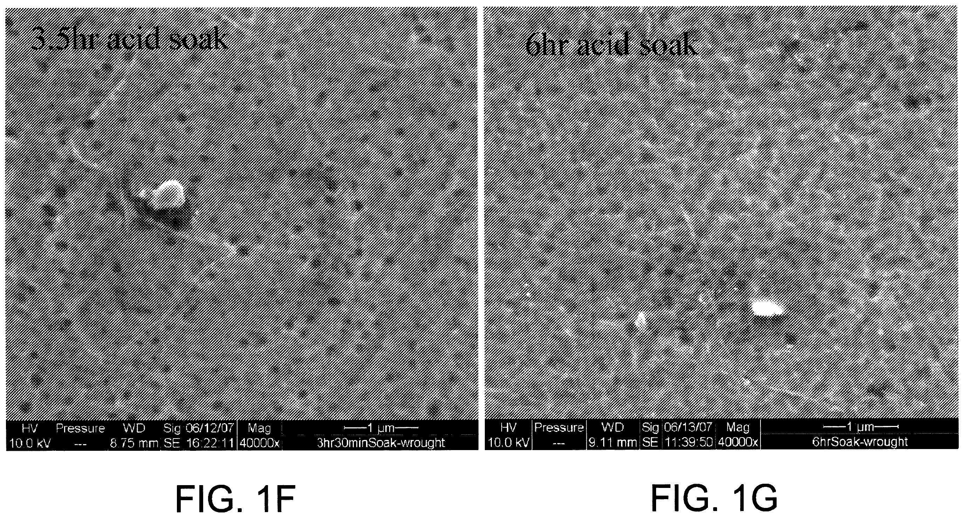

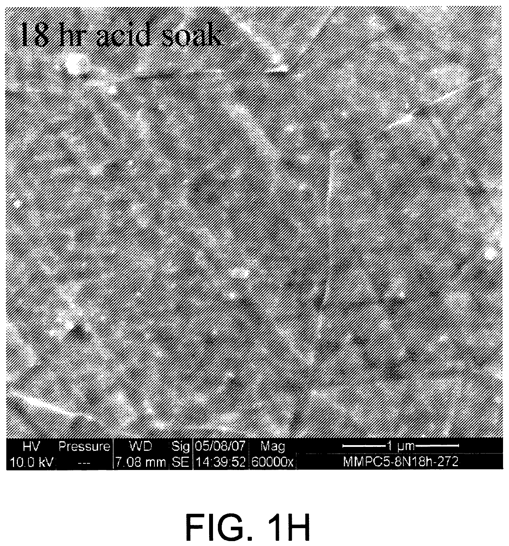

Figure 1 shows the images of a 19 mm (0.75 inch) diameter polished wrought CoCrMo (ASTM F1537) disk and those soaked for 5 min, 30 min, 1 h, 1.5 h, 3.5 h, 6 h, and 18 h in 8N HCl.

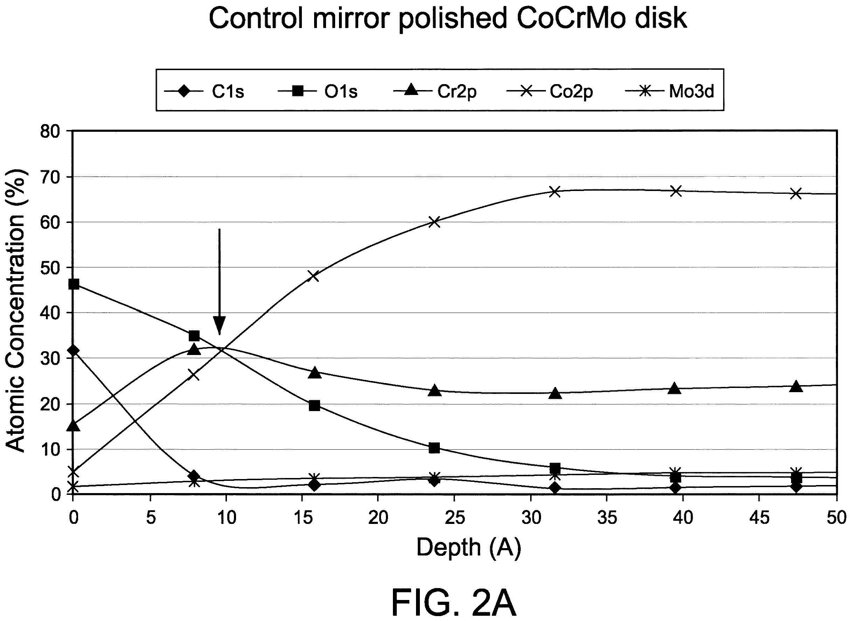

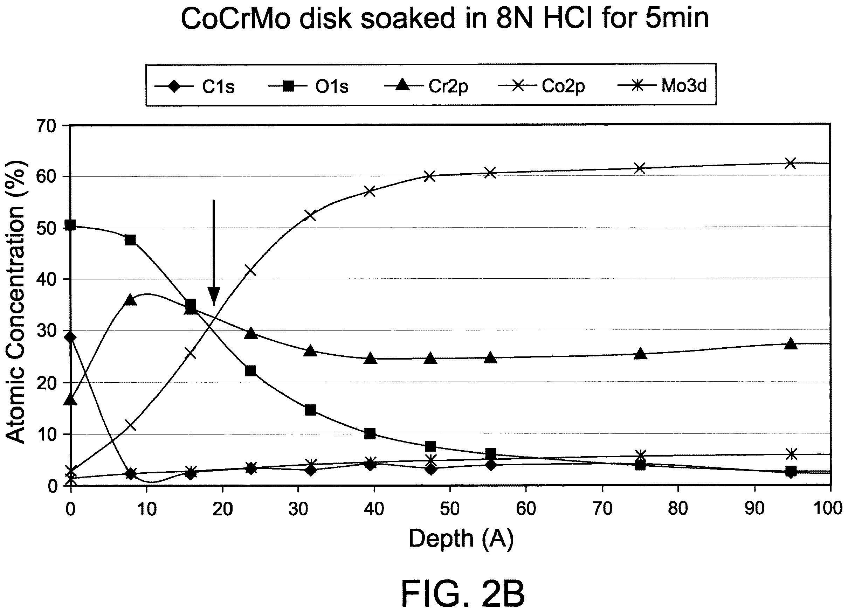

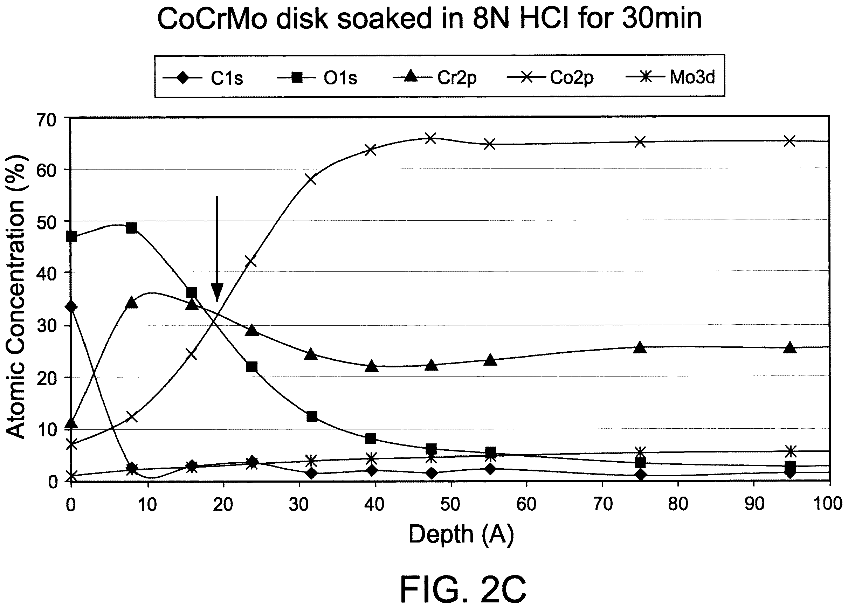

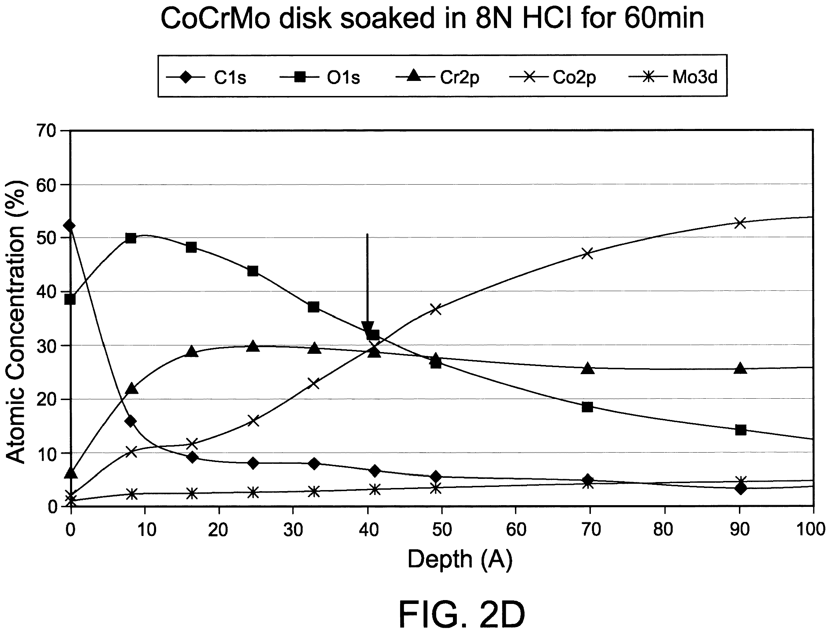

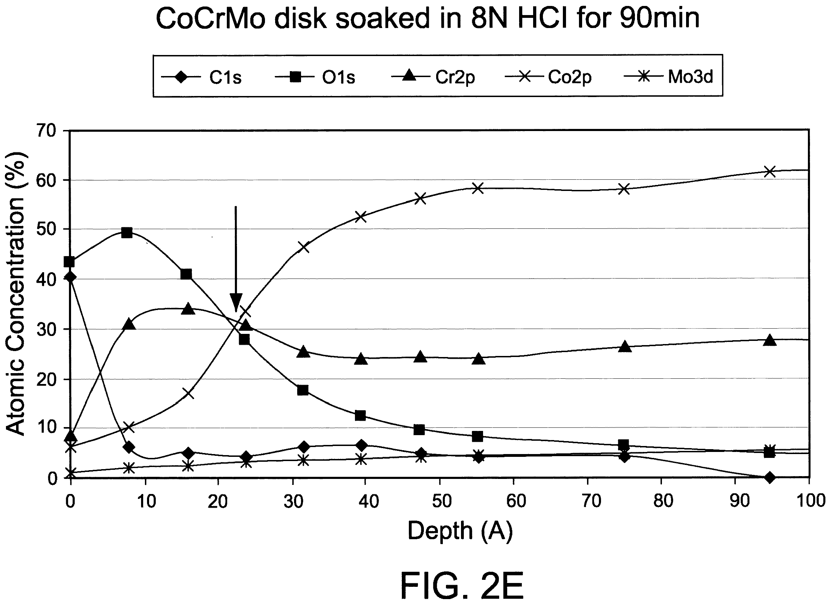

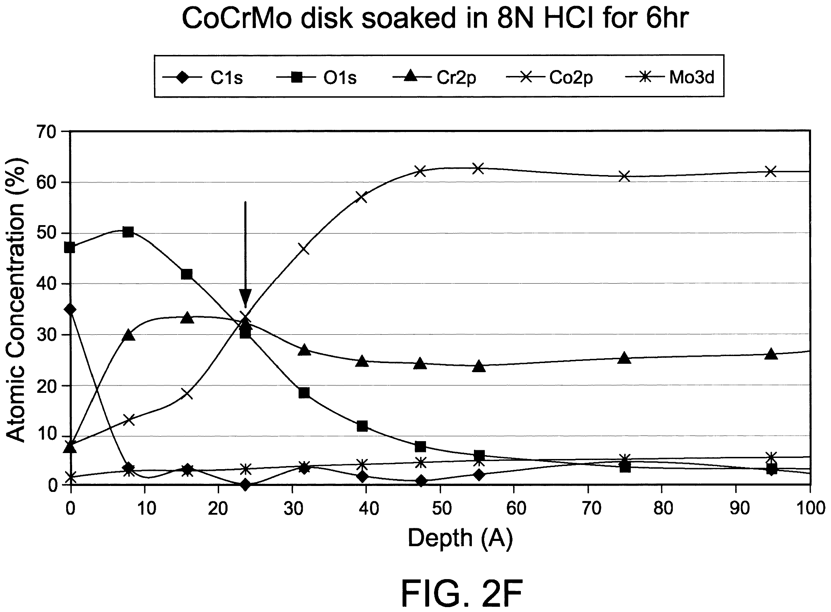

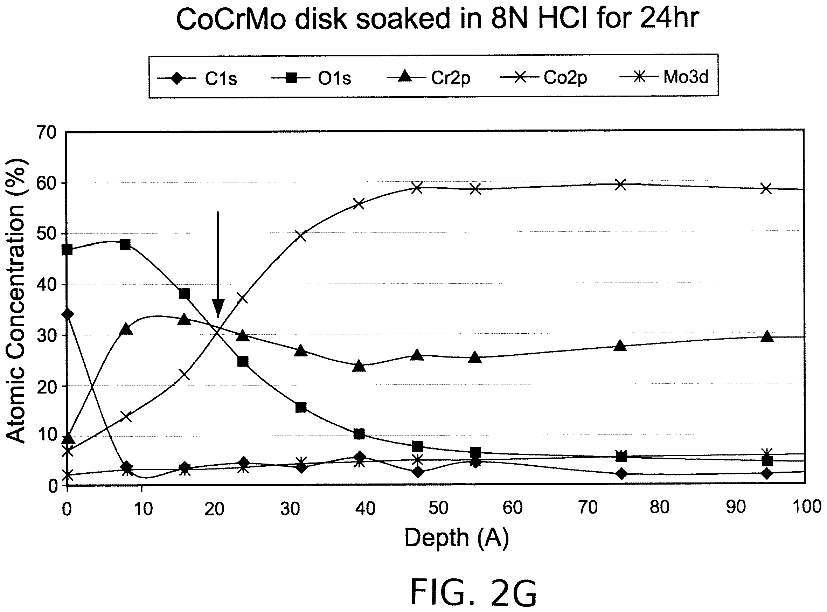

Figure 2 shows the change in depth profile (by XPS) on (A) a polished wrought CoCrMo disk (not soaked in 8N HCl); and after soaking in 8N HCl (room temperature) for (B) 5 min, (C) 30 min, (D) 1 h, (E) 1.5 h; (F) 6 h and (G) 24 h.

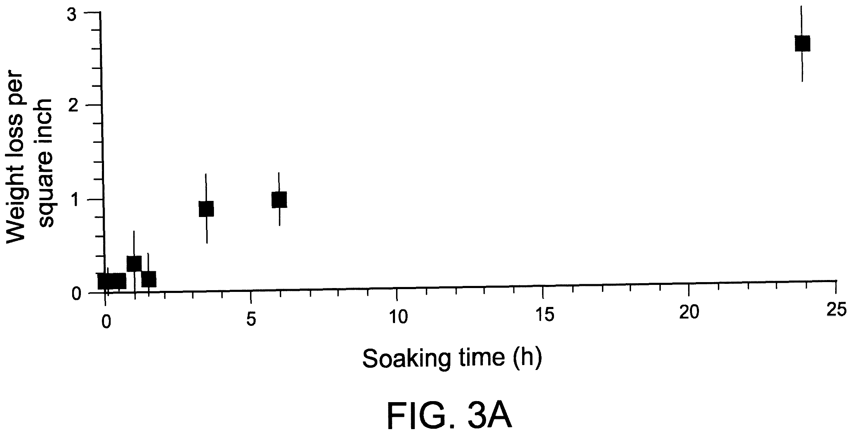

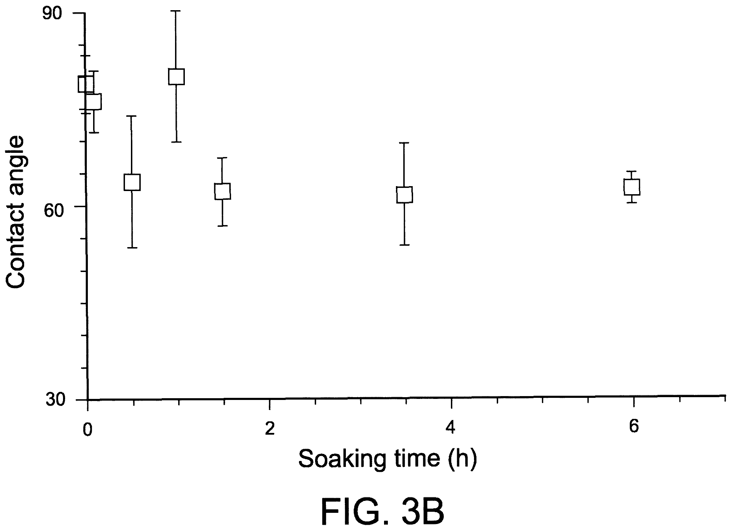

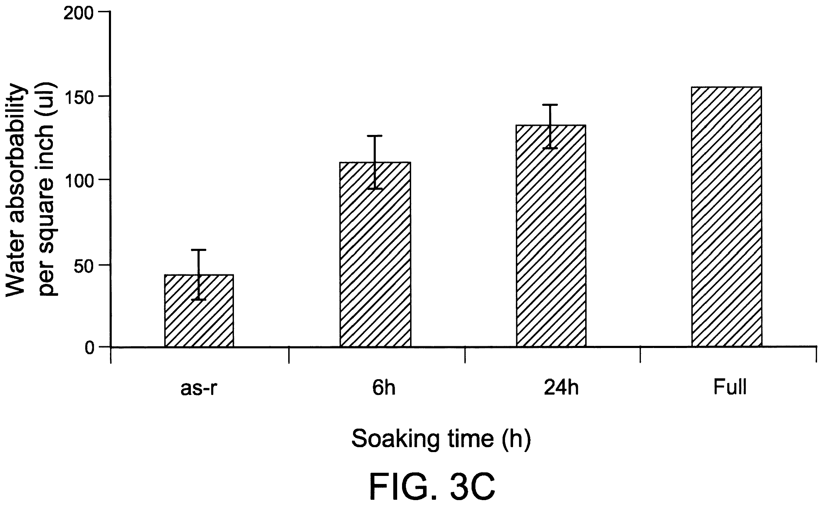

Figure 3 (all disks were treated in 8N HCl for 24 hours at room temperature) shows (A) the weight loss per unit area over acid soaking time on 19 mm (0.75 inch) diameter polished wrought CoCrMo disks, (B) the contact angle of water placed on the surface of the polished wrought disk versus acid soaking time, and (C) the ability to absorb water of the 19 mm (0.75 inch) diameter bead-coated disks versus acid soaking time.

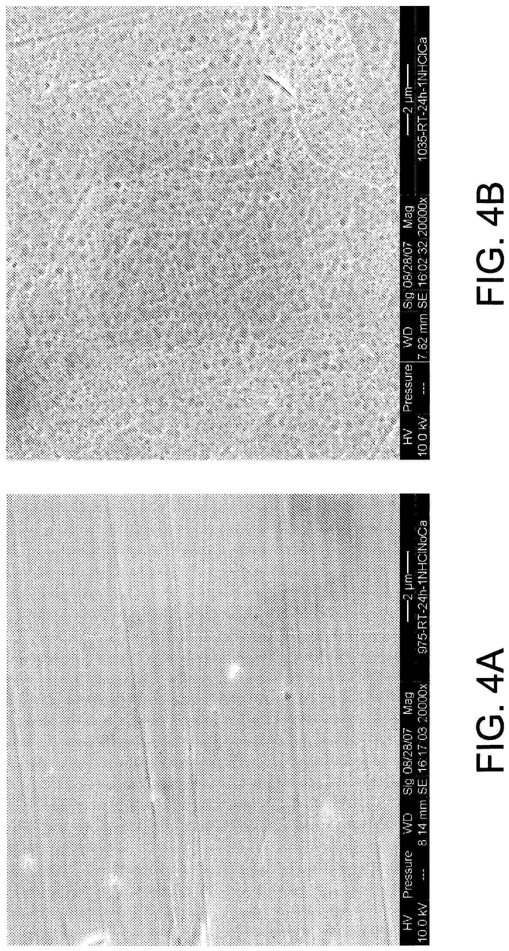

Figure 4 shows (A) the morphologies of a 19 mm (0.75 inch) diameter polished wrought CoCrMo disk after soaking in 1N HCl for 24 hours; and (B) of a 19 mm (0.75 inch) diameter polished wrought CoCrMo disk after soaking in a chloride supplemented 1N HCl for 24 hours. The chloride supplemented 1N HCl is prepared by adding 70 g of CaCl₂.2H₂O in 100 ml of 1N HCl at room temperature. The final concentrations in the chloride supplemented HCl acid are approximately [H⁺] = 0.8N, [Cl⁻] = 8.8N, [Ca²⁺] = 4N

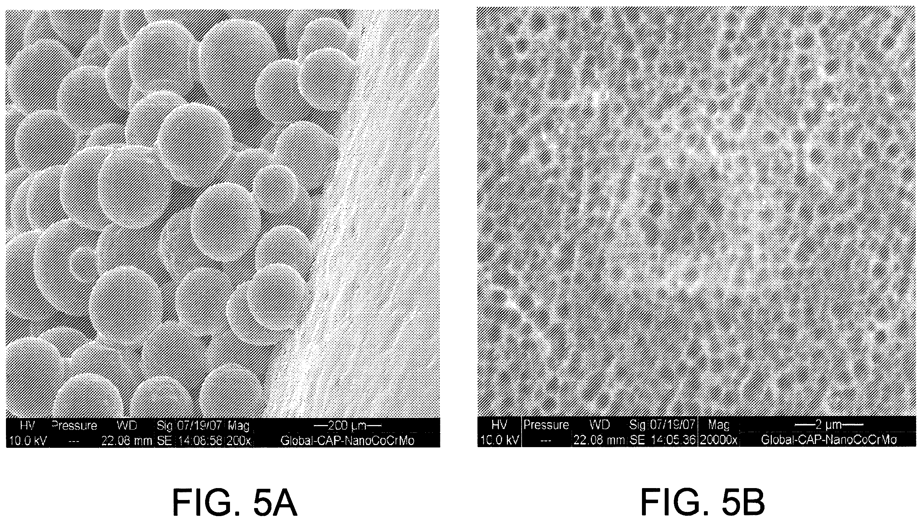

Figure 5 shows (A) a lower magnification image of a component of a shoulder joint prosthesis of the kind sold by DePuy Orthopaedics Inc under the trade mark Global CAP, having a porous surface region provided by sintered beads formed from a CoCrMo alloy (as sold under the trade mark Porocoat) after soaking in acid for 24 hours; (B) a high magnification image of the pits ranging from 100 nm to 500 nm on the bead surface

Surface properties of implant materials have an important role in the adhesion of adjacent cells to the implant. Wettability is one factor that is believed to impact the adhesion to the implant. By using the methods of the instant invention, the surface chemistry of the CoCrMo articles became chromium enriched. In addition, the surface became nanotextured and showed enhanced ability to absorb water. In some embodiments, a 3 to 10 fold improvement in water absorption is observed for the treated implant surfaces versus untreated surfaces. Implants made of such material show improved bone ingrowth.

One aspect of the present invention concerns articles suitable for implantation in a mammal which comprise cobalt-chromium alloy and bear a surface oxide layer that has a thickness of 20 to 40 Å, is enriched in chromium relative to said article, and includes a plurality of indentations that, independently, have a diameter of from about 40 to about 500 nm. In some embodiments, the layer has a liquid absorbing capacity of at least about 0.078 µl.mm⁻² (50 µl.in⁻²).

Liquid absorbing capacity is determined by measuring the amount of water absorbed into the article. The article whose liquid absorbing capacity is to be determined is weighed. Seven drops of 10 µl water which has been purified by reverse osmosis (RO) (a total of seven spots and a total of 70 mg water) are placed on the article. The water is allowed to equilibrate for 5 minutes and then the top surface of the article is contacted by filter paper to remove any water remaining on the bead-coated surface. Then, the weight of the bead-coated disk is measured again. The difference of weight is converted to volume (1mg water = 1 µl). The liquid absorbing capacity is determined by dividing the volume of water by the projected surface area of the article. In some embodiments, the liquid absorbing capacity is reported in µl.mm⁻² (µl.in⁻²).

Some articles of the invention have a liquid absorbing capacity of at least about 0.078 µl.mm⁻² (50 µl.in⁻²). Other articles have a liquid absorbing capacity of at least about 0.116 µl.mm⁻² (75 µl.in⁻²) or at least about 0.156 µl.mm⁻² (100 µl.in⁻²)

If desired, liquid absorbing capacity can be compared to a standard such as DePuy's standard bead-coated disk (having a diameter of 19 mm (0.75 inch), a thickness of 750 µm, and a porosity of 40 to 50%). If the article is highly wettable, the water usually wicks into the article within 5 minutes. If the article is not highly wettable, the water usually beads up on the surface.

Some preferred metal objects include those comprising at least one of cobalt, chromium, and molybdenum, such as wrought CoCrMo. In some articles, the cobalt-chromium alloy is CoCrMo. In some embodiments, the surface of said article comprises cobalt-chromium alloy beads.

Other aspects of the invention concern methods for making nanotextured articles having high wettability. Some articles have properties as described above. Some methods comprise contacting an article that includes cobalt chromium alloy with a solution comprising hydrochloric acid for a time and at a temperature effective to form a surface oxide layer on said article that has a thickness of 20 to 40 Å, is enriched in chromium relative to said article, and includes a plurality of indentations that, independently, have a diameter of from about 40 to about 500 nm. The solution is substantially free of oxidizing agent.

In some processes, the solution has a concentration of H⁺ of at least about 4N and a concentration of Cl⁻ of at least about 4N. The solution can further comprise a chloride-containing compound at a concentration in the range of about 0.01N to 8N. In some embodiments, the solution has a concentration of H⁺ of about 0.8 to about 12N and a concentration of Cl⁻ that is greater than said concentration of H⁺. Preferred chloride-containing compounds include sodium chloride (NaCl), potassium chloride (KCl), calcium chloride (CaCl₂), ammonium chloride (NH₄Cl), ferric chloride (FeCl₃), ferrous chloride (FeCl₂), cobalt chloride (CoCl₂), magnesium chloride (MgCl₂) and mixtures thereof.

The article can be contacted with the solution at any temperature suitable for modifying the surface. In some embodiments, the article is contacted with the solution at a temperature of about 20 to about 100°C, or in some embodiments, at a temperature of about 20 to about 40°C. The article can be contacted with the solution for a time sufficient to provide the desired surface properties. In some processes, for example, the article is contacted with the solution for about 30 minutes to about 96 hours in some processes. In other processes, the article is contacted with the solution for about 1 to about 36 hours.

Typically, articles provided by the invention are suitable for implantation into a mammal. Some articles are joint replacement prostheses or components thereof. Certain articles are components of a hip or knee joint prostheses.

Typically, articles provided by the invention are suitable for implantation into a mammal. Some articles are joint replacement prostheses or components thereof. Certain articles are components of a hip or knee joint prostheses.

The invention is applicable to three-dimensional, porous coatings whihc are provided by beads, which are provided on devices sold by DePuy Orthopaedics Inc under the trade mark Porocoat. In some embodiments, the beads are formed from a CoCrMo material. They can be provided on a substrate using a sintering process.

### EXAMPLES

### The Disks

The mirror polished ASTM F1537 wrought CoCrMo disk, 19 mm (0.75 inch) diameter, was used for weight loss assessment and contact angle measurement. Such disks can be machine cut from a bar stock (ASTM F1537 wrought) and a mirror polished finish (Ra <0.1 µm). The disk can have a coating of spherical CoCrMo beads with sizes ranging approximately from 150 to 250 µm. The coating has a thickness of 750 µm and a diameter of 19 mm (0.75 inch) with a porosity ranging of 40 to 50%. The acid soaking test was carried out at room temperature/condition (18 °C to 23 °C). For a flat 19 mm disk, a volume of at least 30 ml of the acid solution was used; for a 19 mm bead-coated disk, a volume of at least 50 ml of the acid solution was used.

### Acid Solutions

100 ml of 12N HCl was diluted with 50 ml of reverse osmosis (RO) treated water to produce an 8N HCl solution (Solution 1).

100 ml of 12N HCl was diluted with 100 ml of reverse osmosis (RO) treated water to produce a 6N HCl solution (Solution 2).

100 ml of 12N HCl was diluted with 200 ml of reverse osmosis (RO) treated water to produce a 4N HCl solution (Solution 3).

The chloride supplemented 1N HCl is prepared by adding 70 g of CaCl₂.2H₂O in 100 ml of 1N HCl. The final concentrations in the chloride-supplemented hydrochloric acid are approximately [H⁺] = 0.8N, [Cl⁻] = 8.8N, [Ca²⁺] = 4N (Solution 4).

Ultrasound cleaning: after completing each acid soaking time, each of the test sample was rinsed in RO water and followed ultraound cleaning in RO water for 20 minutes, 10 minutes and 5 minutes. The samples were then blown dry with nitrogen and thermally dried at 60°C oven for at least 2 hours.

The etching studies used 19 mm (0.75 inch) polished wrought CoCrMo disks (ASTM F1537) with Ra <0.1 to measure weight loss during acid soaking process. The etching studies used 25.4 mm (1 inch) polished wrought CoCrMo disks (ASTM F1537) to measure contact angles. The etching studies utilized 19 mm (0.75 inch) bead-coated disks (ASTM F1537) to measure the ability to absorb water.

### Example 1

300 ml of Solution 1 was added to a 500 ml beaker. A 19 mm diameter polished wrought CoCrMo disk was added to a fixture formed from PTFE so that only the polished surface was exposed to the acid and allowed to lie vertically. The top of the beaker was sealed with film (sold under the trade mark Parafilm). After soaking for the designated time, the disks were rinsed with running RO water and ultrasound cleaned. The experiment was repeated with 5min, 30 minutes, 1 h, 1.5 h, 3.5 h, 6 h, 18 or 24 h soaking. Micrographs of a control disk (not soaked) and the 5 min, 30 min, 1.5 h, 3.5 h, 6 h and 18 h soaked disks are presented in Figure 1. In Figure 2, the depth profiles of a polished disk and those soaked in 8N HCl for 5min, 30 min, 1 h, 1.5 h, 6 h and 24 h are presented. The depth distribution of Co, Cr, Mo, O, C from the top of the surface was examined by X-ray photoelectron spectroscopy (XPS). Five elements were examined (C1s, O1s, Co2p, Cr2p and Mo3d) at Pass Energy of 224.00 eV and step size 0.4 eV. Using Argon at a sputter setting of 2 kV 2×2, the 200 µm × 200 µm (200u45w15kv) area was sputtered for a total of 2.1 minutes. The sputter cycles included 6 cycles at 0.1 min and 6 cycles at 0.2 min in Alternate Sputter Mode and have a total analysis time of 57 minutes. The depth of the Cr rich layer is defined by the intersection of the Cr and Co depth (indicated by arrows)

Figure 3 shows (A) the weight loss per soaking time, (B) the contact angle of water placed on the surface of the disk versus soaking time, and (C) the ability to absorb water of the bead coated disk surface versus soaking time. The acid soaked bead-coated disks absorbed significantly more water than untreated bead-coated disks. The weight loss is determined by the difference between the disk measured prior to and after soaking. The contact angle is measured by dosing one drop of 5 µl water on the 19 mm (0.75 inch) wrought disk using standard contact angle measuring instrument and extracting the contact angle by applying the drop shape analysis. The same test was repeated for three times. The ability to absorb water is measured as described above.

### Example 2

300 ml of the designated solution was added to a 500 ml beaker. A 19 mm (0.75 inch) polished CoCrMo wrought disk was added to the beaker and allowed to lay flat with the polished surface facing up for 96 hours. The top of the beaker was sealed with film (sold under the trade mark Parafilm). After soaking, the disk was ultrasound cleaned. Disks soaked in 4N or 6N HCl were observed to show color change (slight blue) with disk surface losing its shininess (white cloudy areas appeared on the polished surfaces) but a disk soaked in 2N HCl for four days showed no color change in solution or surface change by appearance.

### Example 3

Two polished 19 mm diameter wrought CoCrMo disks were fixed in a PTFE fixture so that only one surface of each disk was exposed to acid. The two polished disks were soaked in 100 ml of the chloride supplemented HCl acid in a glass container sealed by with a film for 24 hours at room temperature/condition. Another two polished disks were fixed in a PTFE fixture and soaked in 100 ml 1N HCl in a glass container sealed by film for 24 hours at room temperature. Fig. 4A shows the surface after soaked in 1N HCl for 24 hours and Fig. 4B shows the surface after soaked in chloride supplemented 1N HCl

### (solution 4)

The average weight loss per unit area exposed to the acid is 0.45±0.09 mg.cm⁻² (2.9±0.6 mg.in⁻²) for the disks soaked in chloride supplemented HCl acid and it is 0.078±0.15 mg.cm⁻² (0.5±0.2 mg.in⁻²) for the disks soaked in 1N HCl.

### Example 4

A component of a shoulder implant as sold by DePuy Orthopaedics Inc under the trade mark Global CAP (Figure 5A) was soaked in 300 ml 8N HCl solution for 24 hours at room condition in a glass beaker sealed with film. The implant was ultrasound cleaned after removal from the acid solution. Etched pits range from 100 to 500 nm can be observed on part or all of the bead surfaces (Figure 5B).

## Claims

1. A process comprising contacting an article which is formed at least in part from an alloy of cobalt and chromium with a solution which comprises:
(a) hydrochloric acid, and
(b) a chloride containing compound other than hydrochloric acid at a concentration in the range of from 0.01M to 8M,
so that the solution has a concentration of H⁺ of 0.1 to 12M and a concentration of Cl- that is greater than said concentration of H⁺, the solution being free of an additional oxidising agent, the article being contacted with the solution for a time and at a temperature effective to form a surface oxide layer on said article that has a thickness of 20 to 40 Å, is enriched in chromium relative to said article, and includes a plurality of indentations that, independently, have a diameter of from 40 to 500 nm.

2. The process of claim 1, wherein said solution has a concentration of H⁺ of at least 0.1N and a concentration of Cl⁻ of at least 4N.

3. The process of claim 1, wherein said chloride-containing compound is sodium chloride (NaCl), potassium chloride (KCl), calcium chloride (CaCl₂), ammonium chloride (NH₄Cl), ferric chloride (FeCl₃), ferrous chloride (FeCl₂), cobalt chloride (CoCl₂), magnesium chloride (MgCl₂) or a mixture thereof.

4. The process of claim 1, wherein the article is contacted with the solution at a temperature of 20 to 100°C, preferably at a temperature of 20 to 40°C.

5. The process of claim 1, wherein the article is contacted with the solution for 30 minutes to 96 hours.

6. The process of claim 1, wherein said article is a component of a joint prosthesis, preferably a component of a hip or knee joint prosthesis.

## Patentansprüche

1. Verfahren, welches den Vorgang umfasst, einen Artikel, welcher zumindest teilweise aus einer Legierung aus Kobalt und Chrom hergestellt ist, mit einer Lösung in Kontakt zu bringen, welche umfasst:
(a) Chlorwasserstoffsäure und
(b) eine andere Chlorid enthaltende Verbindung als Chlorwasserstoffsäure mit einer Konzentration im Bereich von 0,01 M bis 8 M,
sodass die Lösung eine H⁺-Konzentration von 0,1 bis 12M und eine Cl⁻-Konzentration hat, welche höher als die H⁺-Konzentration ist, wobei die Lösung frei von einem zusätzlichen Oxidationsmittel ist, wobei der Artikel mit der Lösung für eine Zeitdauer und bei einer Temperatur in Kontakt gebracht wird, welche wirkungsvoll sind, um eine Oberflächenoxidschicht auf dem Artikel zu bilden, welche eine Stärke von 20 bis 40 Å hat, relativ zu dem Artikel reicher an Chrom ist und eine Vielzahl von Vertiefungen aufweist, welche, unabhängig voneinander, einen Durchmesser von von 40 bis 500 nm haben.

2. Verfahren nach Anspruch 1, wobei die Lösung eine H⁺-Konzentration von mindestens 0,1N und eine Cl⁻-Konzentration von mindestens 4N hat.

3. Verfahren nach Anspruch 1, wobei die Chlorid enthaltende Verbindung Natriumchlorid (NaCl), Kaliumchlorid (KCl), Kalziumchlorid (CaCl₂), Ammoniumchlorid (NH₄Cl), Eisen(III)-chlorid (FeCl₃), Eisen(II)-chlorid (FeCl₂), Cobaltchlorid (CoCl₂), Magnesiumchlorid (MgCl₂) oder ein Gemisch daraus ist.

4. Verfahren nach Anspruch 1, wobei der Artikel mit der Lösung bei einer Temperatur von 20 bis 100 °C, vorzugsweise bei einer Temperatur von 20 bis 40 °C in Kontakt gebracht wird.

5. Verfahren nach Anspruch 1, wobei der Artikel mit der Lösung für 30 Minuten bis 96 Stunden in Kontakt gebracht wird.

6. Verfahren nach Anspruch 1, wobei der Artikel eine Komponente einer Gelenkprothese, vorzugsweise eine Komponente einer Hüft- oder einer Kniegelenkprothese ist.

## Revendications

1. Procédé comprenant la mise en contact d'un article qui est formé au moins en partie à partir d'un alliage de cobalt et de chrome avec une solution qui comprend :
(a) de l'acide chlorhydrique, et
(b) un chlorure contenant un composé autre que l'acide chlorhydrique à une concentration dans la plage allant de 0,01 M à 8 M,
de sorte que la solution possède une concentration en H⁺ de 0,1 à 12 M et une concentration en Cl⁻ qui est supérieure à ladite concentration en H⁺, la solution étant dépourvue d'un agent oxydant additionnel, l'article étant mis en contact avec la solution pendant une durée et à une température efficace pour former une couche d'oxyde de surface sur ledit article qui a une épaisseur de 20 à 40 Å, est enrichi en chrome par rapport audit article, et comprend une pluralité de dents qui, indépendamment, ont un diamètre de 40 à 500 nm.

2. Procédé selon la revendication 1, dans lequel ladite solution a une concentration en H⁺ d'au moins 0,1 N et une concentration en Cl⁻ d'au moins 4 N.

3. Procédé selon la revendication 1, dans lequel ledit composé contenant du chlorure est le chlorure de sodium (NaCl), le chlorure de potassium (KCl), le chlorure de calcium (CaCl₂), le chlorure d'ammonium (NH₄Cl), le chlorure ferrique (FeCl₃), le chlorure ferreux (FeCl₂), le chlorure de cobalt (CoCl₂), le chlorure de magnésium (MgCl₂) ou un mélange de ceux-ci.

4. Procédé selon la revendication 1, dans lequel l'article est mis en contact avec la solution à une température de 20 à 100 °C, de préférence à une température de 20 à 40 °C.

5. Procédé selon la revendication 1, dans lequel l'article est mis en contact avec la solution pendant 30 minutes à 96 heures.

6. Procédé selon la revendication 1, dans lequel ledit article est un composant d'une prothèse articulaire, de préférence un composant d'une prothèse articulaire de hanche ou de genou.
